## Europäisches Patentamt

## European Patent Office

## Office européen des brevets

(19)

(11) Publication number: **0 028 835**
**B1**

(12) **EUROPEAN PATENT SPECIFICATION**

(45) Date of publication of patent specification: **18.05.83**

(21) Application number: **80106929.5**

(22) Date of filing: **10.11.80**

(51) Int. Cl.³: **C 07 C 9/04, C 07 C 1/04, C 07 C 1/12, C 07 C 1/20, C 07 C 4/00, C 10 G 47/00**

(54) Method of preparing a gas containing a high portion of methane.

(30) Priority: **13.11.79 NL 7908283**

(43) Date of publication of application:
**20.05.81 Bulletin 81/20**

(45) Publication of the grant of the patent:
**18.05.83 Bulletin 83/20**

(84) Designated Contracting States:
**BE DE FR GB NL**

(56) References cited:
DE - A - 2 341 288
GB - A - 733 792
GB - A - 1 080 129
GB - A - 1 198 671
GB - A - 1 265 481
GB - A - 1 437 957
GB - A - 1 443 277
GB - A - 1 462 059
US - A - 3 450 514
US - A - 3 950 368
US - A - 4 079 072

(73) Proprietor: **VEG-GASINSTITUUT N.V.**
**Wilmersdorf 50**
**NL-7327 AC Apeldoorn (NL)**

(72) Inventor: **Takens, Walther**
**Cederlaan 49**
**NL-2282 KC Rijswijk (NL)**
Inventor: **Bottelberghs, Pierre Henri**
**Van Maasdijkstraat 44**
**NL-3555 VP Utrecht (NL)**
Inventor: **Geus, John Wilhelm**
**Gezichtslaan 100**
**NL-3723 GJ Bilthoven (NL)**

(74) Representative: **Türk, Dietmar, Dr. rer. nat. et al,**
**Redies, Redies, Türk & Gille Patentanwälte**
**Brucknerstrasse 20**
**D-4000 Düsseldorf 13 (DE)**

Courier Press, Leamington Spa, England.

## Method of preparing a gas containing a high portion of methane

The invention relates to a method of preparing a gas containing a high portion of methane by methanisation of a gas mixture comprising carbonmonoxide, carbondioxide and hydrogen and reaction with a gas mixture comprising methanol and/or hydrocarbons having up to 20 C-atoms and a portion of less than 20% by weight methane in a reactor.

From DE—AS 1 180 481 (=GB—PS 820 257) a process for the production of gases containing methane is known, wherein the vapour of paraffinic hydrocarbons containing an average of 4—10 carbon atoms and steam are passed through a bed of a nickel catalyst under atmospheric or superatmospheric pressure, the proportion of steam being within the range of 2 to 5 parts by weight for one part by weight of the hydrocarbons or when using hydrocarbons containing an average of 4—7 carbon atoms within the range of 1,5—5 parts by weight for one part by weight of the hydrocarbons. The hydrocarbon vapour and the steam are passed into the catalyst bed at a temperature above 350°C such that the bed is maintained by the reaction at a temperature within the range of 400°C to 550°C. By this process products are obtained having a composition after the removal of steam of about 61— 65% by volume of $CH_4$, 21—23% by volume of $CO_2$, 1% by volume of CO and 12—17% by volume of $H_2$. If a gas having a higher content of methane is desired the methane-rich gas produced in the above-mentioned way may be subjected to the action of a nickel catalyst at a lower temperature, e.g. at 400°C or below in order to bring about the formation of methane by reaction between carbon dioxide, carbon monoxide and hydrogen present in the gas.

For this process a number of nickel catalysts may be used, examples of which can be found in DE—AS 1 199 427 and 1 227 603.

BE—PS 634 920 describes catalysts which are suitable for reforming hydrocarbons with steam and contain nickel or cobalt in the form of the metal or in the form of a compound which may be reduced to form the metal as well as a metal of the platinum group upon a refractory support such as alumina. These catalysts may also contain a small amount of an alkali metal or an earth alkali metal in order to prevent the deposition of carbon upon the catalyst. It is also known from said Belgian patent specification 634 920 that by carrying out the reforming of hydrocarbons having a boiling point of at most 350°C with steam at a temperature of 600—1000°C synthesis gas or a gas having a low methane content are obtained but that by carrying out said process at a temperature of 450—700°C a methane-rich gas is obtained.

According to FR—PS 1 394 202 the life of the catalysts in the process as described in DE—AS 1 180 481 can considerably be increased by recycling hot reaction gases containing water vapour through the catalyst bed mixed with the hydrocarbon vapour and steam being supplied to said bed.

According to the US—PS 3 420 642 the reaction between the hydrocarbons and the steam is carried out in two stages. To the first stage the whole of the steam is supplied but only part of the hydrocarbons. The product gas and undecomposed steam from the first stage are supplied to the second stage together with the remaining portion of the hydrocarbons.

According to DE—AS 1 922 182 the reaction as described is carried out in two reactors. The gas produced in the first reactor and containing methane, hydrogen, carbon monoxide, carbon dioxide and undecomposed steam is cooled by the addition of at least one of the reaction components and the resulting gaseous mixture is introduced into a second reactor in which the components of the mixture react in the presence of a catalyst in such a way that the methane content of the mixture is increased.

According to DE—OS 2 314 804 the conversion of hydrocarbons in the vapour phase having a boiling range up to about 250°C with steam is carried out under a pressure of 15—100 at in the presence of nickel catalysts in two reactors. To the first reactor a mixture of 40—50% by weight of the total amount of preheated hydrocarbons and 80—90% by weight of the total amount of steam is supplied, the resulting product is cooled after having been discharged from the first reactor and then mixed with the remaining portion of preheated hydrocarbons and steam and further converted in a second reactor.

According to NL—PS 296 207 a methane containing gas is prepared by contacting a heated mixture of a hydrocarbon material, a hydrogen containing gas and steam with a very active metal catalyst at a temperature of 450—900°C under a pressure between 3 and 50 at. The hydrogen containing gas may also contain carbon oxides which react with the hydrogen in the presence of the catalysts to form methane. The amount of hydrogen is 0.1—2.5 moles and the amount of steam is 2.5—0.3 moles per atom of carbon in the hydrocarbon material.

According to DE—AS 1 545 462 hydrocarbons with an average number of carbon atoms of 10— 20 are converted with steam and hydrogen to form methane-rich gases. To the mixture of hydrocarbons and steam 0.1—0.8 $Nm^3$ of hydrogen is added for 1 kg of hydrocarbon, so that the hydrogen containing feed mixture needs only be pre-heated to a maximum temperature of 350°C and the reaction can be carried out at a maximum temperature of 450°C. This is favourable because it is generally known that with an increasing preheating temperature of the hydrocarbons endothermal reactions are promoted in the catalyst bed whereby carbon monoxide and hydrogen are formed. This leads to a temperature reduction in the catalyst bed. This reduction of the temperature and the excess

# O 028 835

of carbon monoxide favour the Boudouard reaction according to which carbon dioxide and carbon are formed from carbon monoxide. The carbon formed in this way is, however, deposited upon the catalyst and upon the walls of the reaction vessels.

It is also known that many of the reactions which take place when passing hydrocarbons and steam over a nickel catalyst are exothermal and thus again reduce the formation of methane.

The purpose of the invention is to provide an improved process in which the disadvantages of the processes of the prior art are at least partly avoided.

Accordingly this invention provides a method of preparing a gas containing a high portion of methane by methanisation of a gas mixture comprising carbonmonoxide, carbondioxide and hydrogen and effecting reaction with a gas mixture comprising methanol and/or hydrocarbons having up to 20 C-atoms and a portion of less than 20% by weight methane in a reactor, characterised in that

a) as a gas mixture comprising carbonmonoxide, carbondioxide and hydrogen is used a hydrogen-rich gas containing from 25 to 90% by volume hydrogen,

b) this gas mixture is fed to a first catalyst bed which catalyses the formation of methane, the temperature at the entrance of the catalyst bed being adjusted to from 250 to 550°C,

c) the gas mixture after having left the first catalyst bed is cooled down to the entrance temperature of a second catalyst bed to from 250 to 550°C by addition of a mixture of steam and hydrocarbons the mixture of which has a lower temperature than the entrance temperature of the second catalyst bed,

d) and the gas mixture is fed to a second catalyst bed in which the conversion of hydrocarbons with steam and the other components of the gas mixture is catalysed to form methane.

Preferably the reactor is operated under a pressure of from 0.5 to 10 MPa, more preferably from 1 to 6 MPa and most preferably from 2 to 5 MPa.

According to the invention a hydrogen-rich carbonmonoxide containing gas is forced to react in a reactor over a first catalyst bed to form methane in an exothermic reaction after which the product gas is cooled down by the addition of a hydrocarbon-steam mixture in the same reactor whereupon the resulting mixture is led over a second catalyst bed, to form a methane-rich gas. The gas mixture coming out of the first catalytic bed may still contain so much hydrogen that it protects the catalyst of the second bed, as with the known hydrogasification.

Considering the nature of the catalyst to be used, the first catalyst stimulates the reaction of the carbonmonoxide and carbondioxide with hydrogen to form methane (and water). The second catalyst bed stimulates the conversion of the added hydrocarbons and steam to methane.

As to the catalysts used in the procedure the catalyst in the first bed should be a supported Nickel or Cobalt methanisation catalyst according to the state of the art. This first-bed catalyst in particular should be thermostable and active already at relatively low temperatures; it can be prepared according to DE—OS 1 767 202 but is not limited to this.

Aluminum oxide is preferred as a support for the catalyst.

In the second bed, well-known CRG-type (catalytic rich gas) catalysts can be used but alumina-supported Nickel catalysts based on the above-mentioned preparation procedure (DE—OS 1 767 202) are preferred here too.

Typical for the invention is that the reaction heat of the methanisation reaction, taking place in the first catalyst bed, is being used to bring the reacting mixture at the required entrance temperature of the second catalyst bed. With this the preheating of the reacting mixture outside the reactor can be limited. Due to this the temperature of the added steam/hydrocarbon mixture will be lower than the entrance temperature of the second catalyst bed. The temperatures at the entrance of both catalyst beds can be between 250°C and 550°C. The temperature at the entrance of the first bed will preferably be between 275 and 400°C; at the entrance of the second bed preferably between 300 and 450°C.

The reaction temperatures can be controlled accurately by adding steam to the hydrogen rich gas which is added to the first catalyst bed. It is advisable to feed steam in such an amount that the temperature of the gas mixture when leaving the first catalyst bed is reduced to a temperature not exceeding 800°C, preferably not exceeding 700°C and even more preferably not exceeding 600°C.

The hydrogen-rich gas generally contains:

from 30 to 80% by volume hydrogen
from 5 to 25% by volume carbonmonoxide
from 0 to 25% by volume methane
less than 50% by volume carbondioxide and/or inertgas.

Preferably the hydrogen-rich gas contains:

from 40 to 65% by volume hydrogen
from 12 to 22% by volume carbonmonoxide
from 0 to 10% by volume methane
less than 10% by volume carbondioxide and/or inertgas.

3

It is preferred to use lean gas as hydrogen-rich gas. With lean gas, as described in the prior art, is meant a mixture consisting of $H_2$, CO, $CO_2$, $CH_4$, $H_2O$ and possible inert components with a volume percentage of $H_2$ bigger than the separate volume percentages of CO, $CO_2$ and $CH_4$, but not necessarily bigger than the volume percentage of $H_2O$.

As hydrogen-rich gas which is supplied to the first catalyst bed there can be used a gas mixture obtained by subjecting a gas resulting from coal gasification or hydrocarbon reforming to a subsequent carbonmonoxide shift conversion step and optionally to a carbondioxide removal step.

Preferably the hydrogen-rich gas contains from 2.5 to 5 parts by volume hydrogen per 1 part by volume carbonmonoxide, preferably from 2.9 to 3.2 parts by volume hydrogen per 1 part by volume carbonmonoxide.

The total steam amount required in a certain situation depends upon the hydrogen-rich gas composition, the catalyst-bed entrance temperatures, the operating pressure and the reactor throughput. Insufficient steam addition may lead to carbon deposition in the catalyst bed(s). Due to the number of parameters involved it is difficult to state comprehensively how the change in one of the parameters may affect the required mass input ratios. Obviously the mass input ratios are determined by the thermodynamical conservation laws from which they can be calculated for specific cases. For the procedure according to the invention therefore may be stated the following general mass input ranges:

steam in an amount of 600 to 1200 kg and hydrocarbons in an amount of 300 to 800 kg referred to 1000 m³ gas input to the first catalyst bed ($m_s^3$ is cubic metres at 15°C and .101 MPa).

The mixture of steam and hydrocarbons which is fed to the gas mixture after having left the first catalyst bed has preferably a temperature of from 120 to 300°C, more preferably of from 180°C to 250°C.

The hydrocarbons used in the method of the present invention is preferably the so-called light fraction of hydrocarbons consisting essentially of hydrocarbons having up to 12 C-atoms.

The product gas coming out of the second catalyst bed can still have a relatively high temperature. This product gas can be utilized to heat a mixture of steam and hydrocarbons to a temperature required for the reaction in a third catalyst bed. Although this principle extends to a number of catalyst beds, the thermal profit decreases rapidly, so that the capital costs will then become prohibitive.

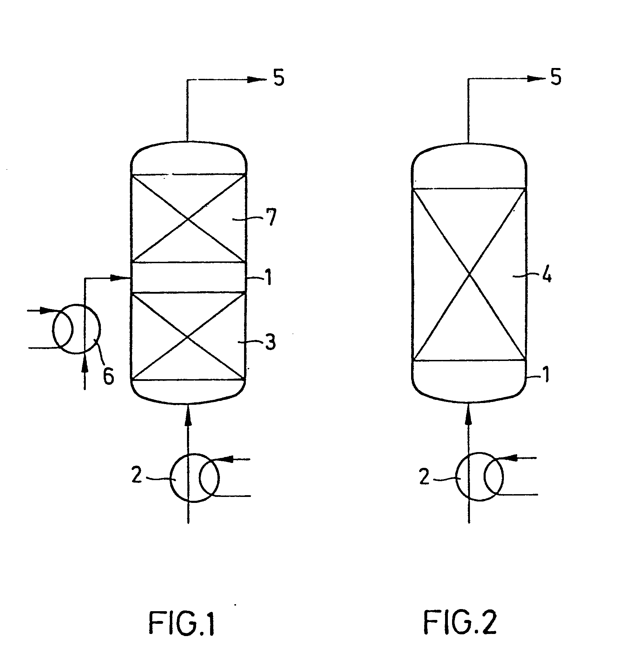

The invention is explained by way of the Figures and examples. Figure 1 schematically represents the method according to the invention, Figure 2 represents the mentioned well-known "hydrogasification". In the Fig. 1 and 2 1 is the reactor, 2 a pre-heater for the reaction mixture, to be fed to 3, the first catalyst bed according to the invention, or 4 according to the well-known method. The discharging of the product gases is performed via a discharge pipe 5. According to the invention a mixture of steam and hydrocarbons is supplied through a pre-heater 6 to the reactor 1 and mixed with the gas coming from the first catalyst bed 3, which mixture is lead into a second catalyst bed 7.

Example 1

In the method of Fig. 1 according to the invention only hydrogen-rich gas is supplied through pre-heater 2. The steam-hydrocarbon mixture is supplied through the pre-heater 6. In the known method of Fig. 2 this happens through pre-heater 2 together with a hydrogen-rich gas.

The composition and quantities of reaction mixture supplied per time-unit are equal in both cases, just as are the pressure and the temperature at the entrance of the first catalyst bed (respectively 3, in Fig. 1 and 4, in Fig. 2). The pressure is 2 MPa.

The compared results are represented in tables 1 and 2.

**0 028 835**

TABLE 1
Survey of results according to methods shown as Fig. 1 and Fig. 2.
In both cases, the quantity of raw materials is identical:
1000 m³ hydrogen-rich gas[*]
1 728 kg of steam of 190°C
1 012 kg of butane of 60°C
The temperature of the mixture of steam and butane is 165°C.

| Type | $Ti^{[+]}$ | $Tm^{[+]}$ | $Tu^{[+]}$ | Calorific value dry product gas | Q in MJ[**] |
|---|---|---|---|---|---|
| Fig. 1 | 300 | 205 | 447 | 28,0 MJ/m³ₛ | 369 |
| Fig. 2 | 300 | — | 480 | 26,9 MJ/m³ₛ | 951 |
| Composition of obtained dry product gas for Fig. 1<br>11,9% $H_2$    0,3% CO    70,3 $CH_4$    17,5% $CO_2$ | | | | | |
| Composition of obtained dry product gas for Fig. 2<br>15,5% $H_2$    0,5% CO    66,2% $CH_4$    17,8% $CO_2$ | | | | | |

[*] The hydrogen-rich gas is of the following composition:
50% $H_2$, 10% CO, 5% $CH_4$, 5% $CO_2$, 30% $H_2O$.
[+] Ti is the temperature at the entrance of the reactor in B and at the entrance of each catalyst bed in A.
Tu is the temperature at the exit of the reactor.
Tm is the temperature of the pre-heated steam/hydrocarbon mixture at A. Temperatures are given in °C.
[**] Q is the necessary preheat for the hydrocarbon-steam mixture.

TABLE 2
Survey of results according to methods shown as Fig. 1 and Fig. 2.
In both cases the quantity of raw materials is identical:
1000 m³ hydrogen rich gas[*]
1 066 kg of steam of 190°C
0 572 kg of butane of 60°C
The temperature of the mixture of steam and butane is 167°C.

| Type | $Ti^{[+]}$ | $Tm^{[+]}$ | $Tu^{[+]}$ | Calorific value dry product gas | Q in MJ[**] |
|---|---|---|---|---|---|
| Fig. 1 | 350 | 205 | 492 | 26,3 MJ/m³ | 213 |
| Fig. 2 | 350 | — | 531 | 24,7 MJ/m³ | 748 |
| Composition of obtained dry pdroduct gas for Fig. 1<br>19,2% $H_2$    0.6% CO    63,3% $CH_4$    16,8% $CO_2$ | | | | | |
| Composition of obtained dry product gas for Fig. 2<br>24,3% $H_2$    1,1% CO    57,4% $CH_4$    17,2% $CO_2$ | | | | | |

[*] The hydrogen-rich gas is of the following composition:
50% $H_2$, 10% CO, 5% $CH_4$, 5% $CO_2$, 30% $H_2O$.
[+] Ti is the temperature at the entrance of the reactor in B and at the entrance at each catalyst bed in A.
Tu is the temperature at the exit of the reactor.
Tm is the temperature of the pre-heated steam/hydrocarbon mixture at Fig. 1. Temperatures are given in °C.
[**] Q is the necessary preheat for the hydrocarbon-steam mixture.

From the examples it appears that at analogous conditions:

1. the performance of Fig. 1 according to the invention delivers a product gas at a higher calorific value at a lower temperature; than known method (Fig. 2);

5

**0 028 835**

2. the reaction mixture of the performance of Fig. 1 according to the invention demands considerably less preheat energy than known method (Fig. 2).

**Claims**

1. Method of preparing a gas containing a high portion of methane by methanisation of a gas mixture comprising carbonmonoxide, carbondioxide and hydrogen and effecting reaction with a gas mixture comprising methanol and/or hydrocarbons having up to 20 C-atoms and a portion of less than 20% by weight methane in a reactor, characterised in that

a) as a gas mixture comprising carbonmonoxide, carbondioxide and hydrogen is used a hydrogen-rich gas containing from 25 to 90% by volume hydrogen,
b) this gas mixture is fed to a first catalyst bed which catalyses the formation of methane, the temperature at the entrance of the catalyst bed being adjusted to from 250 to 550°C,
c) the gas mixture after having left the first catalyst bed is cooled down to the entrance temperature of a second catalyst bed from 250 to 550°C by addition of a mixture of steam and hydrocarbons the mixture of which has a lower temperature than the entrance temperature of the second catalyst bed,
d) and the gas mixture is fed to a second catalyst bed in which the conversion of hydrocarbons with steam and the other components of the gas mixture is catalysed to form methane.

2. Method according to claim 1, characterised in that the reactor is operated under a pressure from 0.5 to 10 MPa.
3. Method according to claim 2, characterised in that the reactor is operated under a pressure from 1 to 6 MPa.
4. Method according to claim 3, characterised in that the reactor is operated under a pressure from 2 to 5 MPa.
5. Method according to any of claims 1 to 4, characterised in that the temperature at the entrance of the first catalyst bed is adjusted to from 275 to 400°C.
6. Method according to any of claims 1 to 5, characterised in that the temperature at the entrance of the second catalyst bed is adjusted to from 300 to 450°C.
7. Method according to any of claims 1 to 6, characterised in that the hydrogen-rich gas contains

from 30 to 80% by volume hydrogen
from 5 to 25% by volume carbonmonoxide
from 0 to 25% by volume methane
less than 50% by volume carbondioxide and/or inertgas.

8. Method according to claim 7, characterised in that the hydrogen-rich gas contains

from 40 to 65% by volume hydrogen
from 12 to 22% by volume carbonmonoxide
from 0 to 10% by volume methane
less than 10% by volume carbondioxide and/or inertgas.

9. Method according to claim 7, characterised in that the hydrogen-rich gas is lean gas.
10. Method according to any of claims 1 to 9, characterised in that the hydrogen-rich gas contains from 2.5 to 5 parts by volume hydrogen per 1 part by volume carbonmonoxide.
11. Method according to claim 10, characterised in that the hydrogen-rich gas contains from 2.9 to 3.2 parts by volume hydrogen per 1 part by volume carbonmonoxide.
12. Method according to any of claims 1 to 11, characterised in that the temperature of the gas mixture when leaving the first catalyst bed is reduced to a temperature not exceeding 800°C by feeding steam to the first catalyst bed.
13. Method according to claim 12, characterised in that the temperature of the gas mixture when leaving the first catalyst bed is reduced to a temperature not exceeding 700°C.
14. Method according to claim 13, characterised in that the temperature of the gas mixture when leaving the first catalyst bed is reduced to a temperature not exceeding 600°C.
15. Method according to any of claims 1 to 14, characterised in that the mixture of steam and hydrocarbons which is fed to the gas mixture after having left the first catalyst bed has a temperature of from 120 to 300°C.
16. Method according to claim 15, characterised in that the mixture of steam and hydrocarbons which is fed to the gas mixture after having left the first catalyst bed has a temperature from 180 to 250°C.
17. Method according to any of claims 1 to 16, characterised in that in step c) there are added

6

steam in an amount of 600 to 1200 kg and hydrocarbons in an amount of 300 to 800 kg per each 1000 m³$_s$ gas input to the first catalyst bed.

18. Method according to any of claims 1 to 17, characterised in that the hydrogen-rich gas which is supplied to the first catalyst bed is obtained by subjecting a gas, resulting from coal gasification or hydrocarbon reforming, to a subsequent carbonmonoxide shift conversion step and optionally to a carbondioxide removal step.

## Patentansprüche

1. Verfahren zur Herstellung eines Gases mit großem Methangehalt durch Methanisieren eines Gasgemisches enthaltend Kohlenmonoxid, Kohlendioxid und Wasserstoff und Umsetzung mit einem Gasgemisch entahltend Methanol und/oder Kohlenwasserstoffe mit bis zu 20 C-Atomen und einem Anteil von weniger als 20 Gew.% Methan in einem Reaktor, dadurch gekennzeichnet, daß

a) als Gasgemisch enthaltend Kohlenmonoxid, Kohlendioxid und Wasserstoff ein wasserstoffreiches Gas enthaltend 25 bis 90 Vol.% Wasserstoff verwendet wird,

b) dieses Gasgemisch in ein erstes Katalysatorbett geleitet wird, das die Bildung von Methan katalysiert, obei die Temperatur am Eingang des Katalysatorbetts auf 250 bis 550°C eingestellt ist,

c) das Gasgemisch nach dem Verlassen des ersten Katalysatorbettes durch Zugabe eines Gemisches aus Dampf und Kohlenwasserstoffen mit einer Temperatur, die unter der Eintrittstemperatur des zweiten Katalysatorbettes liegt, auf eine Eintrittstemperatur von 250 bis 550°C eines zweiten Katalysatorbettes abgekühlt wird,

d) und das Gasgemisch in ein zweites Katalysatorbett geleitet wird, in dem die Umwandlung von Kohlenwasserstoffen mit Dampf und den anderen Komponenten des Gasgemisches unter Bildung von Methan katalysiert wird.

2. Verfahren nach Anspruch 1, dadurch gekennzeichnet, daß der Reaktor bei einem Druck von 0.5 bis 10 MPa betrieben wird.

3. Verfahren nach Anspruch 2, dadurch gekennzeichnet, daß der Reaktor bei einem Druck von 1 bis 6 MPa betrieben wird.

4. Verfahren nach Anspruch 3, dadurch gekennzeichnet, daß der Reaktor bei einem Druck von 2 bis 5 MPa betrieben wird.

5. Verfahren nach einem oder mehreren der Ansprüche 1 bis 4, dadurch gekennzeichnet, daß die Temperatur beim Eingang in das erste Katalysatorbett auf 275 bis 400°C eingestellt wird.

6. Verfahren nach einem oder mehreren der Ansprüche 1 bis 5, dadurch gekennzeichnet, daß die Temperatur am Eingang in das zweite Katalysatorbett auf 300 bis 450°C eingestellt wird.

7. Verfahren nach einem oder mehreren der Ansprüche 1 bis 6, dadurch gekennzeichnet, daß das wasserstoffreiche Gas enthält

30 bis 80 Vol.% Wasserstoff
5 bis 25 Vol.% Kohlenmonoxid
0 bis 25 Vol.% Methan
weniger als 50 Vol.% Kohlendioxid und/oder Inertgas.

8. Verfahren nach Anspruch 7, dadurch gekennzeichnet, daß das wasserstoffreiche Gas enthält

40 bis 65 Vol.% Wasserstoff
12 bis 22 Vol.% Kohlenmonoxid
0 bis 10 Vol.% Methan
weniger als 10 Vol.% Kohlendioxid und/oder Inertgas.

9. Verfahren nach Anspruch 7, dadurch gekennzeichnet, daß das wasserstoffreiche Gas Schwachgas ist.

10. Verfahren nach einem oder mehreren der Ansprüche 1 bis 9, dadurch gekennzeichnet, daß das wasserstoffreiche Gas 2.5 bis 5 Vol.Teile Wasserstoff pro 1 Vol.Teil Kohlenmonoxid enthält.

11. Verfahren nach Anspruch 10, dadurch gekennzeichnet, daß das wasserstoffreiche Gas 2.9 bis 3.2 Vol.Teile Wasserstoff pro 1 Vol.Teil Kohlenmonoxid enthält.

12. Verfahren nach einem oder mehreren der Ansprüche 1 bis 11, dadurch gekennzeichnet, daß die Temperatur des das erste Katalysatorbett verlassenden Gasgemisches durch Zugabe von Dampf in das erste Katalysatorbett auf eine Temperatur herabgesetzt wird, die 800°C nicht übersteigt.

13. Verfahren nach Anspruch 12, dadurch gekennzeichnet, daß die Temperatur des das erste Katalysatorbett verlassenden Gasgemisches auf eine Temperatur herabgesetzt wird, die 700°C nicht übersteigt.

14. Verfahren nach Anspruch 13, dadurch gekennzeichnet, daß die Temperatur des das erste

**O 028 835**

Katalysatorbett verlassenden Gasgemisches auf eine Temperatur herabgesetzt wird, die 600°C nicht übersteigt.

15. Verfahren nach einem oder mehreren der Ansprüche 1 bis 14, dadurch gekennzeichnet, daß das Gemisch aus Dampf und Kohlenwasserstoffen, das dem das erste Katalysatorbett verlassenden Gasgemisch zugeführt wird, eine Temperatur von 120 bis 300°C aufweist.

16. Verfahren nach Anspruch 15, dadurch gekennzeichnet, daß das Gemisch aus Dampf und Kohlenwasserstoffen, das dem das erste Katalysatorbett verlassenden Gasgemisch zugeführt wird, eine Temperatur von 180 bis 250°C aufweist.

17. Verfahren nach einem oder mehreren der Ansprüche 1 bis 16, dadurch gekennzeichnet, daß in der Stufe c) Dampf in einer Menge von 600 bis 1200 kg und Kohlenwasserstoffe in einer Menge von 300 bis 800 kg pro jeweils 1000 m³ Gas, das in das erste Katalysatorbett eingeleitet wird, zugeführt wird.

18. Verfahren nach einem oder mehreren der Ansprüche 1 bis 17, dadurch gekennzeichnet, daß das wasserstoffreiche Gas, das in das erste Katalysatorbett eingeleitet wird, dadurch erhalten wird, daß ein Gas aus der Kohlevergasung oder der Kohlenwasserstoff-Reformierung einer anschließenden Kohlenmonoxid-Shift-Umwandlungsstufe und gegebenenfalls einer Kohlendioxid-Entfernungsstufe unterworfen wird.

### Revendications

1. Procédé de préparation d'un gaz contenant une haute proportion de méthane par méthanisation d'un mélange de gaz contenant du monoxyde de carbone, du dioxyde de carbone et de l'hydrogène et en effectuant une réaction avec un mélange de gaz comprenant du méthanol, et/ou des hydrocarbures ayant jusqu'à 20 atomes de C, et une partie de moins de 20% en poids de méthane, dans un réacteur, caractérisé par le fait que:

a) comme mélange de gaz comprenant du monoxyde de carbone, du dioxyde de carbone et de l'hydrogène est utilisé un gaz riche en hydrogène contenant de 25 à 90% en volume d'hydrogène,

b) ce mélange de gaz est alimenté vers un premier lit de catalyse qui catalyse la formation de méthane, la température à l'entrée du lit de catalyse étant ajustée de façon à être comprise entre 250 et 550°C,

c) le mélange de gaz, après avoir quitté le premier lit de catalyse, est refroidi jusqu'à la température d'entrée d'un second lit de catalyse, comprise entre 250 et 550°C, par addition d'un mélange de vapeur et d'hydrocarbures, dont le mélange présente une température plus basse que la température d'entrée du second lit de catalyse,

d) et le mélange de gaz est alimenté vers un second lit de catalyse dans lequel la conversion des hydrocarbures avec de la vapeur et les autres composants du mélange de gaz est catalysée pour former du méthane.

2. Procédé selon la revendication 1, caractérisé par le fait que l'on fait fonctionner le réacteur sous une pression comprise entre 0,5 et 10 MPa.

3. Procédé selon la revendication 2, caractérisé par le fait que l'on fait fonctionner le réacteur sous une pression comprise entre 1 et 6 MPa.

4. Procédé selon la revendication 3, caractérisé par le fait que l'on fait fonctionner le réacteur sous une pression comprise entre 2 et 5 MPa.

5. Procédé selon l'une des revendications 1 à 4, caractérisé par le fait que la température à l'entrée du premier lit de catalyse est adjustée pour être comprise entre 275 et 400°C.

6. Procédé selon l'une des revendications 1 à 5, caractérisé par le fait que la température à l'entrée du second lit de catalyse est adjustée pour être comprise entre 300 et 450°C.

7. Procédé selon l'une des revendications 1 à 6, caractérisé par le fait que le gaz riche en hydrogène contient:

— de 30 à 80% en volume d'hydrogène
— de 5 à 25% en volume de monoxyde de carbone
— de 0 à 25% en volume de méthane
— moins de 50% en volume de dioxyde de carbone et/ou d'un gaz inerte.

8. Procédé selon la revendication 7, caractérisé par le fait que le gaz riche en hydrogène contient:

— de 40 à 65% en volume d'hydrogène
— de 12 à 22% en volume de monoxyde de carbone
— de 0 à 10% en volume de méthane
— moins de 10% en volume de dioxyde de carbone et/ou d'un gaz inerte.

9. Procédé selon la revendication 7, caractérisé par le fait que le gaz riche en hydrogène est du gaz "pauvre".

10. Procédé selon l'une des revendications 1 à 9, caractérisé par le fait que le gaz riche en hydrogène contient entre 2,5 et parties en volume d'hydrogène pour 1 partie en volume de monoxyde de carbone.

11. Procédé selon la revendication 10, caractérisé par le fait que le gaz riche en hydrogène contient entre 2,9 et 3,2 parties en volume d'hydrogène pour 1 partie en volume de monoxyde de carbone.

12. Procédé selon l'une des revendications 1 à 11, caractérisé par le fait que la température du mélange de gaz lorsqu'il quitte le premier lit de catalyse est ramenée à une valeur ne dépassant pas 800°C, en alimentant de la vapeur dans le premier lit de catalyse.

13. Procédé selon la revendication 12, caractérisé par le fait que la température du mélange de gaz lorsqu'il quitte le premier lit de catalyse est ramenée à une valeur ne dépassant pas 700°C.

14. Procédé selon la revendication 13, caractérisé par le fait que la température du mélange de gaz lorsqu'il quitte le premier lit de catalyse est ramenée à une valeur ne dépassant pas 600°C.

15. Procédé selon l'une des revendications 1 à 14, caractérisé par le fait que la température du mélange de vapeur et d'hydrocarbures qui est ajouté au mélange de gaz après que celui-ci ait quitté le premier lit de catalyse est comprise entre 120 et 300°C.

16. Procédé selon la revendication 15, caractérisé par le fait que la température de mélange de vapeur et d'hydrocarbures qui est ajouté au mélange de gaz après que celui-ci ait quitté le premier lit de catalyse est comprise entre 180 et 250°C.

17. Procédé selon l'une des revendications 1 à 16, caractérisé par le fait que, dans l'étape c), pour chaque 1000 $m_s^3$ de gaz à l'entrée du premier lit de catalyse, on ajoute une quantité de vapeur comprise entre 600 et 1200 kg, et une quantité d'hydrocarbures comprise entre 300 et 800 kg.

18. Procédé selon l'une des revendications 1 à 17, caractérisé par le fait que le gaz riche en hydrogène qui alimente le premier lit de catalyse est obtenu en soumettant un gaz résultant d'une gazéification au charbon ou du reformage d'hydrocarbures, à une étape de conversion par substitution par le monoxyde de carbone et éventuellement à une étape d'élimination du dioxyde de carbone.

FIG.1

FIG.2